# NEW EUROPEAN PATENT SPECIFICATION

(11) **EP 0 089 335 B2**
(45) Date of publication and mention of the opposition decision: **10.03.1993**
(45) Mention of the grant of the patent: 30.11.1988
(21) Application number: 81902679.0
(22) Date of filing: 18.09.1981
(51) Int. Cl.: A61F 2/16

(54) **POSTERIOR CHAMBER INTRA-OCULAR TRANSPLANT DEVICE**
IMPLANTAT FÜR DIE POSTERIORE AUGENKAMMER
DISPOSITIF DE TRANSPLANTATION INTRA-OCULAIRE DANS LA CHAMBRE POSTERIEURE

(43) Date of publication of application: 28.09.1983
(73) Proprietor: Federal Patent Corporation, Miami, Florida 33181-2597 (US)
(72) Inventor: Clayman, Henry Mark, Miami, FL 33181-2597 (US)
(74) Representative: Laight, Martin Harvey
(86) International application number: US8101256
(87) International publication number: WO8300998

(56) References cited:
- FR-A- 1 103 399
- GB-A- 2 046 099
- US-A- 3 866 249
- US-A- 3 913 148
- US-A- 3 913 148
- US-A- 4 056 855
- US-A- 4 159 546
- US-A- 4 174 543
- US-A- 4 242 760
- US-A- 4 251 887
- SU,A, USSR Inventor's Certificate No. 563,174 Published 22 July 1977 ALEKSEEV
- "Experience With Twelve Cases Of Intraoclar Anterior Chamber Implants For Aphakia" by J. Boberg-Ans,British Journal of Ophthalmology, Vol.45, No.1, Issued January 1961, pages 37-43.
- Med. J. 1979, Vol 5, pages 64 to 74, S. Shearing "Complications of Shearing posterior chamber intraocular lens"
- Pseudophakos, The C. V. Mosby Company, Saint Louis 1978, N. S. Jaffe et al, page 38, figure 4-3
- Intraocular lenses, pages 314 and 315
- Norme Française NF S 90-750

## Description

### Background and summary of the invention

The invention relates to an intra-ocular implantable device.

Surgical removal or opaque lenses from the eyes of cataract patients is one of the most common surgical procedures. After such surgery, contact lenses or spectacles are usually prescribed to provide at least limited vision for the patient. While spectacle lenses and contact lenses have a number of optical drawbacks, the ready ability to remove or replace them if defective makes them safe and attractive appliances.

For some patients, however, spectacles and contact lenses are not workable. Many older patients are unable to insert and remove the contact lenses or even handle the thick spectacles. The same is true with very young patients. Fortunately for such patients a surgical alternative is available-implantation of a prosthetic or artificial lens into the interior of the eye to replace the opaque natural lens which has been surgically removed. While this type of surgery for all cataract patients may not be appropriate now, improvements in lenses and techniques, such as the present invention, may make such surgery universal for cataract patients in the future. This operation is called lens implant insertion and well over 100,000 such operations have taken place to date in the United States.

The first intra-ocular lens of modern times was implanted by Ridley in 1949 into the posterior chamber of an eye. Because of difficulties encountered with fixation in the posterior chamber, most implantations thereafter were in the anterior chamber or involved clipping in some fashion to the iris. Pseudo- phakos, by Jaffe, Galin, Hirschman, and Clayman (the latter, the present inventor) (C. V. Mosby Company 1978), summarizes the developments in the field and also describes many of the different types of devices which have been implanted in the past.

Although contrary to early efforts, placement of a device in the posterior chamber has a number of substantial advantages. Accordingly, in recent years several devices have been proposed and developed for placement in that chamber. The Shearing intra-ocular lens, which is described in U.S. Patent4,159,546, uses a pair of spring-like loops on either side of the lens (optic) which are positioned in the area between the iris and the ciliary body known as the ciliary sulcus. The Shearing lens is piano-convex with the plane surface designed to contact the posterior capsule or surface of the natural lens, which membrane is normally left intact but often perforated (discissed), during extracapsular cataract extraction (ECCE). The optic portion of the Shearing lens is circular. The Shearing lens is similar in construction to an earlier device of Barraquer, but which was designed for placement in the anterior chamber. US 4242760, US 4073014, US 4080709, US 3913148, and UK 2046099A disclose other examples of posterior chamber devices.

The present invention relates to a unique implantable intra-ocular device having a number of substantial advantages over prior art devices, including the device of Shearing. As in Shearing, in the present invention two haptic loops are attached to a lens, opposite each other. However, the lens of the present invention has a vertical dimension (ie. height) greater than its horizontal dimension (ie. width) such that the length of the incision required to insert the lens is minimized. The experience has been that when an implantable device displaces from its desired position, it tends to move downwards or if the pupil is asymetric the latter tends to peak upwards. Since this decentering tends to be movement in the vertical direction, making that dimension greater helps ensure that the lens will be maintained in a position to satisactorily carry out its function.

GB-A-204699 discloses an intra-ocular device in which the lens is essentially circular but in which diametrically opposed peripheral appertures are provided for receiving an instrument to handle the lens. Although the provision of said apertures marginally reduces the width of the lens, the lens width remains substantially the same as the lens height.

According to the present invention, there is provided an intra-ocular device for implantation in the posterior chamber of an eye comprising a lens optic for providing the optical function of the device, the lens optic having upper and lower compressible haptic loops attached thereto on opposing ends thereof, for contacting the eye surface in the posterior cham- berto position the lens optic in the posterior chamber, the compressibility of the haptic loops providing centering of the lens optic in position behind the pupillary aperture, characterised in that the lens optic is ovoid shaped having a height greater than its maximum width such that the length of the incision required to insert the device is minimized and such that if vertical decentering occurs acceptable optical function is enhanced, in comparison with a circular lens optic, by occupying the pupillary aperture with the ovoid shaped lens optic.

As in Shearing, both of the haptic loops are compressible.

However, in contract to Shearing, the lower loop preferably has a stiffness greater than that of the upper loop. This may be achieved by using dissimilar thicknesses in the upper and lower loops. Providing the lower haptic loop with a greater stiffness ensure that the device will remain in a proper position, and the optic will occupy all or most of the pupillary aperture. Although the lens of the invention is designed for fixation in the ciliary sulcus, it also may be suitable for insertion in the capsular "bag" (ie. the remnant of the capsule of the natural lens, after extracapsular cataract extraction).

Implantation can either take place at the time the cataract is removed, i.e., primary implantation, ordur- ing a second operation subsequent thereto, i.e., secondary implantation. As noted above, during ECCE the posterior capsule or surface of the natural lens is usually left intact, but is often perforated to guard against the possibility that it will later become opaque. If that occurs the perforation will allow a distinct image to impinge on the retina to provide satisfactoryvi- sion. This step of perforation is called discission. During primary implantation the normal procedure is to carry out discission after t he implanted lens has been put in place. After the nucleus and cortex of the natural lens has been removed, the pressure of the vitreous fluid behind the natural lens capsule often pushes that posterior surface forward. In the Shearing lens the membrane presses directly againstthe plane rear surface of the lens, which can make discission a difficult operation without decentering the lens and/or getting vitreous fluid into the anterior chamber.

This problem however, is much reduced by a preferred embodiment of the present invention in which the rear surface of the lens is convex, providing sufficient room to carry out discission without difficulty. In addition, the optical characteristics of the lens of the present invention are believed to be improved by making the rear surface convex and the front surface planar.

According to another aspect of the present invention, the front surface of the lens is provided with at least one, and preferably upper and lower, angulated bores which extend into the lens at an angle to the vertical of, for example, 15° to 20°. In the past it has been conventional to insert the device into the eye using forceps. However, opening forceps in the limited space within the anterior chamber can be disadvantageous to the corneal endothelium. By providing angulated bores, the implant can be fixated at either the upper or lower angulated bore with either a fine spatula or forceps and maneuvered interiorly into position.

According to another aspect of the invention, the lens is provided with straight line external portions in the lateral edges of the optic. These straight edges not only minimize the incision required to insert the implant but also guide the lens into position as it is inserted. Lenses having a circular shape, such as the Shearing lens, may have some tendency to go off center during insertion, as their maximum diameter clears the incision internally.

To implant the device of the present invention, an air bubble (or other substance to deform the anterior chamber) is usually placed in the anterior chamber and the device slid into the chamber behind the bubble through a corneal-scleral incision. The device is then moved inferiorly so that the lower loop compresses in the inferior (lower) ciliary sulcus. Inferior movement can then be continued with the lower loop compressed until the upper loop clears the pupillary margin. If necessary to clear the margin, the upper loop can also be compressed using a second instrument, or the upper iris can be retracted. When the implant is released, the device springs into place and will be held without suturing, (though a suture can be used through the upper loop). An angulated bore is preferably provided in the lens for permitting ready manipulation of the lens within the eye with a fine spatula orother instrument. The device of the present invention can be implanted with relative ease by either a right or left-handed surgeon.

Other objects and purposes of the invention will become clear from the following non-limiting detailed description of the drawings.

### Brief description of the drawings

Figure 1 shows a front view of the device of the present invention.
Figure 2 shows a side view of the device of the present invention.
Figure 3 shows a schematic view of the device of the present invention being inserted into the anterior chamber through an appropriate incision, after an extracapsular cataract extraction.
Figure 4 shows a further schematic view of the present invention with the lower haptic loop being compressed in the inferior ciliary sulcus by a spatula or similar instrument engaging the upper angulated bore.
Figure 5 shows a schematic view of the present invention with the implant moved inferiorly behind the pupillary margin.
Figure 6 shows a schematic view of the present invention with the implant sprung into the desired position behind the iris with the loops in the ciliary sulcus and the discission being carried out behind the lens.

### Detailed description of the drawings

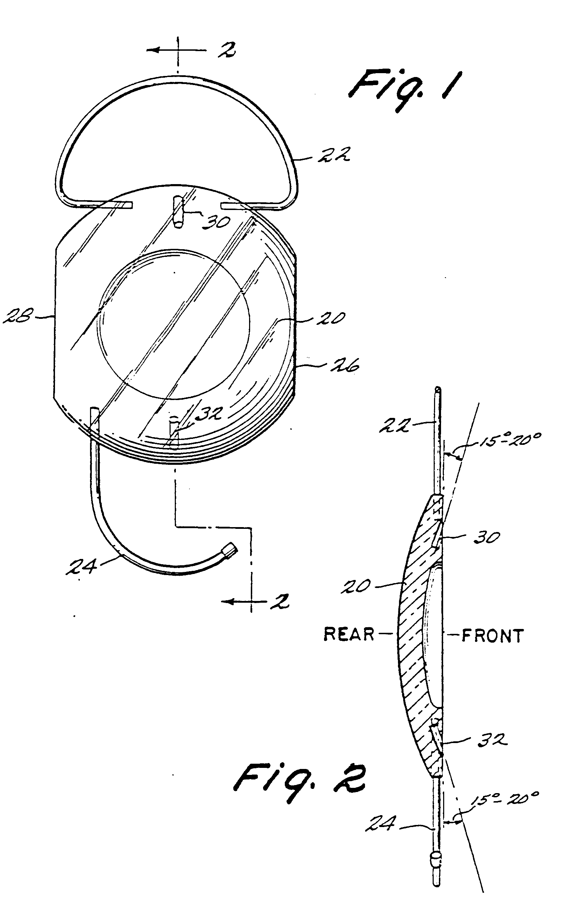

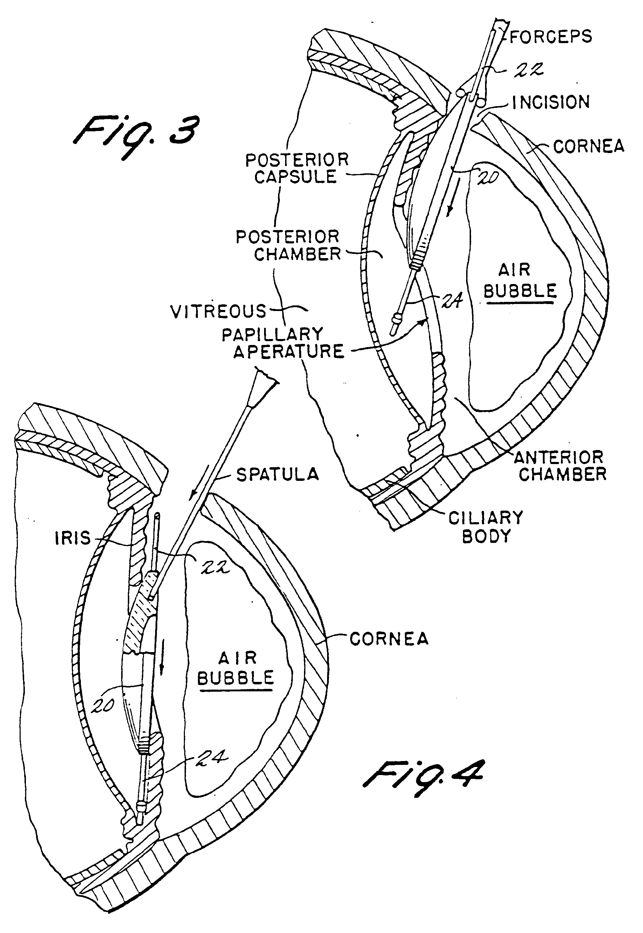

Reference is now made to Figure 1 and to Figure 2 which show respectively front and side views of the present invention. The device of the present invention is comprised of an ovoid shaped lens 20 of suitable material, upper haptic loop 22 attached to lens 20 and lower haptic loop 24 also attached to lens 20. The two haptic loops may be inserted into drill holes or molded with the optic and are fixed in place at the periphery of lens 20 opposite each other. The edge of the lens between the two loops includes straight lines portions 26 and 28 which, as noted above, aid in guiding the lens into position through a small incision. The lower haptic loop 24 acts like a spring during insertion of the device as explained in detail below. Angulated bores 30 and 32 are provided in the front surface of lens 20 for fixating the device to a fine spatula or other similar instrument to properly insert and position the lens, avoiding trauma to the corneal endothelium. An angle of 15° to 20° to the vertical is believed satisfactory.

The lower loop 24 has a greater stiffness than upper loop 22 to ensure the lens centers after insertion. This differential stiffness can, for example, be provided by making the upper loop of 5-0 polypropylene or nylon and the lower loop of 4-0 polypropylene or nylon or other bio-acceptable material. Since displacement after implantation when it occurs is usually vertical, acceptable optical function is also enhanced by making the lens ovoid with a vertical dimension greater than its horizontal dimension, thus occupying the pupillary aperture with the lens optic.

Upper loop 22 is preferably closed by being fixed at both ends, unlike the Shearing device, to have the greater strength which results from two points of fixation. In addition, closed upper loop 22 can be sutured in place, if the surgeon desires.

To aid in discission, the rear optic surface of the lens 20 which contacts the capsule which is the rear surface of the natural lens of the eye is convex rather than piano. The front surface of the optic is essentially piano and this piano-convex configuration is believed to have superior optical characteristics.

Lens 20 is preferably formed of a suitable plastic such as polymethylmethacrylate or glass and may be made either by injection or compression molding or lathe cutting or any other techniques (or a combination) using conventional techniques for making lenses for intra-ocular implantation.

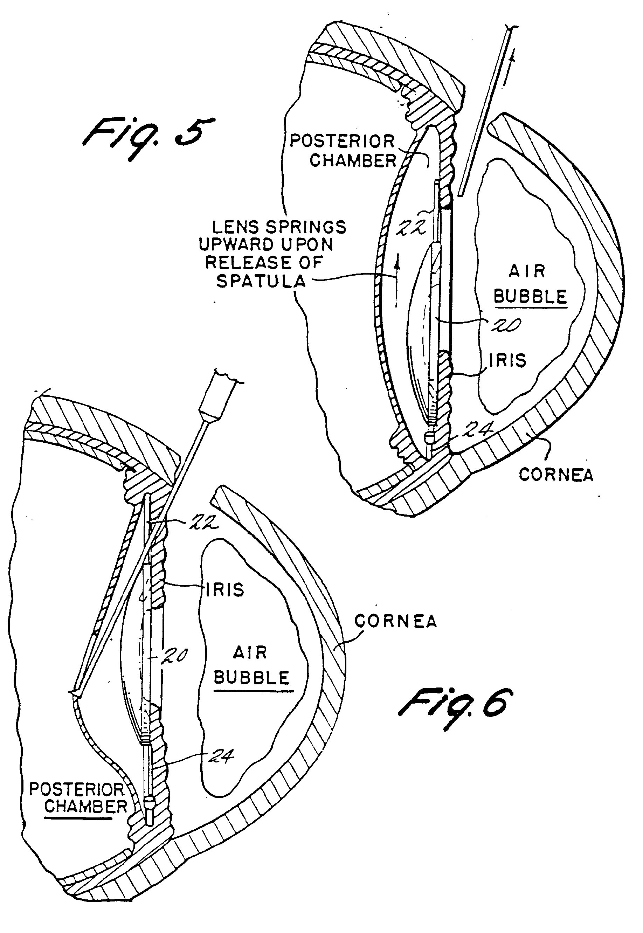

Figures 3 through 6 show schematically the surgical procedure for inserting the present device. Referring to the Figure 3, the device of the present invention is slipped through the incision into the anterior chamber by a suitable forceps under an air bubble which has been previously provided. Referring to Figure 4, the implant is then fixated at the upper angulated bore hole 30 with a fine spatula or other similar instrument and maneuvered inferiorly until the lower loop 24 compresses against the inferior ciliary sulcus in the posterior chamber. The lower bore hole permits insertion upside down from the illustrated and preferred technique if desired by the surgeon. As shown in Figure 4, lower loop 24 is compressed until the upper loop 22 clears the pupillary margin. If necessary, the upper loop can also be compressed by a second instrument to clear the pupillary margin and/or the iris can be retracted. The implant can then be released as shown in Figure 5 to spring behind the iris due to the resiliency inherent in the lower loop 24, the forces applied by the spatula and the balloting effect of the air bubble. The spatula can then be withdrawn. After the implant has sprung into the desired position behind the iris and in the ciliary sulcus, the pupil can be constricted by suitable means and,a peripheral iridect- omy (removal of part of the iris) may be performed. If desired, the upper loop can be sutured to provide a fixed anchor. At this point as shown in Figure 6 a discission may be performed to obviate late opacification of the posterior capsule. The wound can now be sutured and the procedure terminated.

Many changes in modification in the above described embodiment of the invention can, of course, be carried outwithout departing from the scope of the invention defined in the following claims.

## Claims

1. An intra-ocular device for implantation in the posterior chamber of an eye comprising a lens optic (20) for providing the optical function of the device, the lens optic (20) having upper and lower compressible haptic loops (22, 24) attached thereto on opposing ends thereof, for contacting the eye surface in the posterior chamber to position the lens optic (20) in the posterior chamber, the compressibility of the haptic loops (22, 24) providing centering of the lens optic (20) in position behind the pupillary aperture
characterised in that the lens optic (20) is ovoid shaped having a height greater than its maximum width such that the length of the incision required to insert the device is minimized and such that if vertical decentering occurs acceptable optical function is enhanced, in comparison with a circular lens optic, by occupying the pupillary aperture with the ovoid shaped lens optic (20).

2. A device as claimed in Claim 1, wherein the periphery of the lens has the shape of a circle from which opposed segments have been removed.

3. A device as claimed in any one of the preceding claims, wherein the front surface of the lens has at least one bore extending at an angle to the vertical dimension of said lens of 15 to 20° for fixating upon a spatula or similar instrument during implantation in an eye.

4. A device as claimed in any one of the preceding claims, wherein said upper loop is fixed to the lens at both ends and said lower loop is fixed to the lens at only one end.

5. A device as claimed in any one of the preceding claims, wherein the lower loop has a stiffness greater than the upper loop.

6. A device as claimed in any one of the preceding claims, wherein the lens is piano-convex, the rear surface being convex and the front surface being planar.

7. A device as claimed in Claim 1, wherein:-
upper and lower angulated bore holes are provided adjacent the upper and lower edges of the lens, each hole extending at acute angle to the vertical;
the lens has a convex rear surface; and
the lower loop has a stiffness greater than that of the upper loop for maintaining said lens in position after implantation.

## Patentansprüche

1. Intra-okulare Einrichtung zur Implantation in die hintere Kammer eines Auges mit einer optischen Linse (20) zur Bereitstellung der optischen Funktion der Einrichtung, wobei die optische Linse (20) obere und untere zusammendrückbare haptische Schlaufen (22, 24) aufweist, die an deren gegenüberliegenden Enden angebracht sind und zum Kontaktieren der Augenwand in der hinteren Kammer dienen, um die optische Linse (20) in der hinteren Kammer zu positionieren, wobei die Zusammendrückbarkeit der haptischen Schlaufen ein Zentrieren der optischen Linse (20) hinter der Pupillenöffnung bewirkt,
dadurch gekennzeichnet, daß die optische Linse (20) eiförmig ist und eine Höhe hat, die größer als ihre maximale Breite ist, so daß die Länge des zum Einsetzen der Einrichtung erforderlichen Einschnitts minimiert ist und daß, wenn eine senkrechte Dezentrierung eintritt, die akzeptable optische Funktion durch Belegen der Pupillenöffnung mit der eiförmigen optischen Linse (20) verstärkt wird im Vergleich zu einer kreisförmigen optischen Linse.

2. Einrichtung nach Anspruch 1, wobei der Umfang der Linse die Form eines Kreises hat, von dem gegenüberliegende Abschnitte entfernt wurden.

3. Einrichtung nach einem der vorhergehenden Ansprüche, wobei die Vorderfläche der Linse mindestens eine Bohrung aufweist, die sich unter einem Winkel von 15 bis 20° zur Vertikalerstreckung der Linse erstreckt, um einen Spatel oder ein ähnliches Instrument während der Implantation in ein Auge einzusetzen.

4. Einrichtung nach einem der vorhergehenden Ansprüche, wobei die obere Schlaufe an der Linse an beiden Enden befestigt ist und die untere Schlaufe an der Linse nur an einem Ende befestigt ist.

5. Einrichtung nach einem der vorhergehenden Ansprüche, wobei die untere Schlaufe eine größere Steifheit als die obere Schlaufe hat.

6. Einrichtung nach einem der vorhergehenden Ansprüche, wobei die Linse plankonvex, die Rückseite konvex und die Vorderseite plan ist.

7. Einrichtung nach Anspruch 1, wobei:
die oberen und unteren im Winkel stehenden Bohrlöcher in der Nähe des oberen und unteren Randes der Linse angeordnet sind, wobei sich jedes Loch unter einem spitzen Winkel zur Vertikalen erstreckt;
die Linse eine konvexe Rückseite hat; und
die untere Schlaufe eine größere Steifheit als die obere Schlaufe besitzt, um die Linse nach der Implantation in Position zu halten.

## Revendications

1. Dispositif intra-oculaire à implanter dans la chambre postérieure d'un oeil, comprenant un élément optique à lentille (20) destiné à établir la fonction optique du dispositif, élément optique (20) à des extrémités opposées duquel sont fixées des boudes haptiques compressibles supérieure et inférieure (22, 24) pour entrer en contact avec la surface de l'oeil dans la chambre postérieure afin de positionner l'élément optique à lentille (20) dans la chambre postérieure, la compressibilité des boucles haptiques (22, 24) réalisant le centrage de l'élément optique à lentille (20) en position derrière l'ouverture pupillaire,
caractérisé en ce que l'élément optique à lentille (20) est de forme ovoïde ayant une hauteur supérieure à sa largeur maximale afin que la longueur de l'incision demandée pour insérer le dispositif soit minimisée et afin que, si un décentrement vertical apparaît, une fonction optique acceptable soit améliorée, en comparaison avec un élément optique à lentille circulaire, par occupation de l'ouverture pupillaire par l'élément optique à lentille (20) de forme ovoïde.

2. Dispositif selon la revendication 1, dans lequel la périphérie de la lentille présente la forme d'un cercle dont des segments opposés ont été supprimés.

3. Dispositif selon l'une quelconque des revendications précédentes, dans lequel la surface avant de la lentille présente au moins un trou formant avec la dimension verticale de ladite lentille un angle de 15 à 20° pour la fixation sur une spatule ou un instrument similaire durant l'implantation dans un oeil.

4. Dispositif selon l'une quelconque des revendications précédentes, dans lequel ladite boucle supérieure est fixée à la lentille par les deux extrémités et ladite boucle inférieure est fixée à la lentille par une seule extrémité.

5. Dispositif selon l'une quelconque des revendications précédentes, dans lequel la boucle inférieure possède une rigidité plus grande que celle de la boucle supérieure.

6. Dispositif selon l'une quelconque des revendications précédentes, dans lequel la lentille est plan- convexe, la surface arrière étant convexe et la surface avant étant plane.

7. Dispositif selon la revendication 1, dans lequel :
des trous obliques supérieur et inférieur sont prévus à proximité immédiate des bords supérieur et inférieur de la lentille, chaque trou formant un angle aigu avec la verticale ;
la lentille présente une surface arrière convexe ; et
la boucle inférieure présente une rigidité plus grande que celle de la boucle supérieure afin de maintenir ladite lentille en position après l'implantation.
